(19) Europäisches Patentamt
European Patent Office
Office européen des brevets

(11) Veröffentlichungsnummer: **0 171 397 B1**

(12) # EUROPÄISCHE PATENTSCHRIFT

(45) Veröffentlichungstag der Patentschrift: **21.02.90**

(51) Int. Cl.⁵: **A 61 K 35/78**, A 61 K 31/44

(21) Anmeldenummer: **85900614.0**

(22) Anmeldetag: **28.01.85**

(86) Internationale Anmeldenummer: **PCT/CH 85/00013**

(87) Internationale Veröffentlichungsnummer: **WO 85/03442 (15.08.85** Gazette **85/18)**

(54) **THERAPEUTISCHES MITTEL ZUR BEKÄMPFUNG VON DURCH HERPESVIREN VERURSACHTEN INFEKTIONEN.**

(30) Priorität: **02.02.84 CH 484/84**

(43) Veröffentlichungstag der Anmeldung: **19.02.86 Patentblatt 86/8**

(45) Bekanntmachung des Hinweises auf die Patenterteilung: **21.02.90 Patentblatt 90/8**

(84) Benannte Vertragsstaaten: **FR**

(56) Entgegenhaltungen:
**Handbook of chemistry and Physics, 52 Auflage, 1971, S. E44**
**Pharmazeutische Stoffliste, 7 Auflage.ABDA, Frankfurt/Main, S. 283-288**
**Merck Index, 8 Auflage, 1935, S 118**
**Ullmanns, Bd, 7, 4. Auflage, Verlag Chemie, Weinheim, (1980), S.161**
**aAnnales Pharmaceutiques Francaises Band 35,1977,nr.9-10 M Amoros et al "Propriétés anti-viraies de quelques extraits de plantes indigènes" S 371-376**
**Chem Abstracts Band 87 nr.11 12.9.87. Columbus Ohio L V Lozyuk "Anti-viral properties of some compounds of plant origin" S. 50**

**Die Akte enthält technische Angaben, die nach dem Eingang der Anmeldung eingereicht wurden und die nicht in dieser Patentschrift enthalten sind.**

(73) Patentinhaber: **BUPHARM AG, Binningerstrasse 11, CH-4103 Bottmingen (CH)**

(72) Erfinder: **BÜCHNER, Stanislaw, Antoni, Astershagstrasse 13, CH-4103 Bottmingen (CH)**
Erfinder: **BÜCHNER, Paul, Binningerstrasse 11, CH-4103 Bottmingen (CH)**

(74) Vertreter: **Patentanwälte Schaad, Balass & Partner, Dufourstrasse 101 Postfach, CH-8034 Zürich (CH)**

**EP 0 171 397 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung eines Extraktes zur Herstellung eines therapeutischen Mittels zur Bekämpfung von durch Herpesviren verursachten Infektionen.

Zur Bekämpfung von durch Herpesviren verursachten Infektionen werden u.a. Thymidinanaloge (Jod-Desoxyuridin), Vidarabin, Tromantadin und neuerdings Acyclovir verwendet, die jedoch, in bezug auf ihre Wirksamkeit, nicht voll zu befriedigen vermögen.

Aufgabe der vorliegenden Erfindung war es, ein wirksames therapeutisches Mittel zur Bekämpfung von durch Herpesviren verursachten Infektionen zu entwickeln, das auch über längere Zeiträume problemlos eingesetzt werden kann.

Es wurde nun überraschenderweise gefunden, dass diese Aufgabe mit einer Wirkstoffkombination, die in der Pflanze Chelidonium majus enthalten ist und die mit Wasser, einem niederen Alkohol oder einem Gemisch solcher Lösungsmittel extrahiert wird, gelöst wird.

Chelidonium majus L. ist eine Vertreterin der Papaveroidea, einer Unterfamilie der Papaveraceae und ist in weiten Teilen Europas und Asiens als wildwachsende Pflanze verbreitet.

In der Volksheilkunde gilt diese Pflanze, mit dem deutschen Namen Schöllkraut, als ein häufig verwendetes Leber- und Gallenmittel. Da sich im Milchsaft neben Alkaloiden proteolytische Enzyme befinden, wurde der aus dem Kraut frisch austretende Milchsaft auch zur Beseitigung von Warzen benutzt. Herba Chelidonii wurde vor allem als Spasmolytikum bei mit Spasmen einhergehenden Erkrankungen des Magen-Darm-Traktes, einschliesslich Gallensteinleiden und Gallenblasenentzündung (Cholezystopathie und Choletithiasis), eingesetzt (Hänsel und Haas, Therapie mit Phytopharmaka, Springer-Verlag 1983).

Von M. Amoros et al., Annales pharmaceutiques françaises, 1977, 35, no. 9-10, S. 371-376 wurden verschiedene Extrakte von Pflanzen in vitro auf ihre antivirale Wirksamkeit untersucht. Die Versuche mit dem alkoholischen Extrakt von Chelidonium majus verliefen negativ, d.h. es wurde keine virale Aktivität angezeigt.

Chelidonium majus enthält neben geringen Mengen ätherischen Öls mehrere Alkaloide. Der Alkaloidgehalt liegt im Kraut zwischen 0,012 und 0,6 Gew.-% und in den Wurzeln zwischen 0,2 bis 1,4 Gew.-%. Diese Alkaloide können wie folgt in drei Gruppen eingeteilt werden: Protopin-, Protoberberin- und Benzophenanthridin-Typus. Zum ersten Typus gehören α- und β-Allokryptopin und Protopin, zum zweiten Berberin und zum dritten Chelidonin, α-Homochelidonin, Chelerythrin und Sanguinarin, wobei letztere in erster Linie als Wirkstoffe der Pflanze gelten.

In der neueren Literatur werden von Steinegger und Hänsel Chelidonium, Lehrbuch der Pharmakognosie (Springer-Verlag 1972) und Zepernick, Langhammer, Lüdcke, Lexikon der offiziellen Arzneipflanzen (Walter de Gruyter, 1984) die cholagogen Wirkungen der Chelidonium-Alkaloide dem Berberin, im Sinne eines die glatte Muskulatur erregenden cholezystokinetischen Effektes, und dem Chelidonin nur die morphinähnliche analgetische und die spasmolytische Wirkung auf die glatte Muskulatur zugesprochen. Mehrere Präparate mit dieser Indikation sind klinisch auf die Wirksamkeit bei Lebererkrankungen getestet worden.

Das Gerüst der Benzophenanthridin-Alkaloide weist ein tetrazyklisches Ringsystem auf, das aus einem Naphthalinkern besteht, dem ein Isochinolinkern ankondensiert ist. Die beiden zentralen Ringe sind bei einigen Vertretern aromatisch, bei anderen hydriert. Das Stickstoffatom ist stets methyliert. Alkaloide dieses Bauplanes sind in ihrer Verbreitung auf die Tribus der Chelidoneae innerhalb der Papaveraceae beschränkt. Sie kommen hier aber nie allein vor, sondern stets begleitet von Protopin und Alkaloiden des Protoberberin-Typus.

Die genannten Hauptalkaloide des Chelidonium majus vom Benzophenanthridin-Typus weisen folgende Eigenschaften auf:

Chelidonin wirkt morphinähnlich, zentralberuhigend und analgetisch. Es zeigt eine spasmolytische Wirkung auf die glatte Muskulatur, ähnlich wie Papaverin, reicht in der Wirkungsstärke aber keineswegs an Morphin oder Papaverin heran. Chelidonin ist kristallinisch, löslich in Wasser und unlöslich in Alkohol. Sein Schmelzpunkt wird in der Literatur mit 133 bis 134°C bzw. 135 bis 136°C und sein UV λ max. mit 238 nm, 289 nm bzw. 239 nm, 289 nm angegeben.

Chelerythrin bewirkt in grösseren Dosen Muskelstarre und hemmt das Atemzentrum. Sein Schmelzpunkt wird in der Literatur mit 267 bis 269°C bzw. 269 bis 270°C und sein UV λ max. mit 227 nm, 282 nm bzw. 228 nm, 282 nm, 319 nm angegeben.

Sanguinarin wirkt narkotisch und bewirkt, in grösseren Dosen örtlich angewendet, die Lähmung der sensiblen Nervenendigungen. Sein Schmelzpunkt wird in der Literatur mit 243 bis 244°C bzw. 242 bis 243°C und sein UV λ max. mit 234 nm, 284 nm, 324 nm bzw. 234 nm, 284 nm, 325 nm angegeben.

Bodalski et al berichten in Dissertationes Pharmaceut. 9, 157 (1957) über eine hemmende Wirkung der Alkaloide Chelerythrin und Sanguinarin auf die Bakteriophagen $1_{017}$ und $T_2$ in einer Konzentration von 1:1000 und auf Bacterium coli in einer Konzentration von 1:10 000 bis 1:100 000. Nach Stickel, Arch. der Pharmazie 262, 488 und Bodalski et al loc. cit. zeigen Chelidonine, Berberine und ein Gemisch aus Chelerythrin und Sanguinarin eine ausgeprägte antibakterielle Wirkung. Maske und Holmes (Chemistry and Physiology New York, 1952) haben gefunden, dass Chelidonium-Alkaloide die Zellteilung im Mitosestadium hemmen. Ausserdem wird den Chelidonium-Alkaloiden eine gewisse onkostatische Aktivität zugeschrieben (Sokoloff et al, Growth 28, 225 bis 231, 1964).

Gemäss der Feststellung der Erfinder ist die in der Pflanze Chelidonium majus enthaltene Wirkstoffkombination zur Bekämpfung von durch Herpesviren, wie Herpes zoster-Varizellen-Virus und Herpes simplex, verursachten Infektionen geeignet; sie wirkt insbesondere zur Verkürzung der Heilzeit. Diese Wirkstoffkombination wird deshalb erfindungsgemäss mit gutem Erfolg vor allem zur Bekämpfung von

durch diese DNA-Virusarten verursachten Erkrankungen, wie Herpes zoster, Herpes simplex Typ 1 und Typ 2, Gingivostomatitis herpetica, Herpes genitalis und Keratoconjunctivis herpetica, topisch oder systemisch eingesetzt. Dieser Einsatz kann in der Verwendung der Pflanze bzw. seiner Teile oder von aus der Pflanze, einschliesslich ausgewählter Teile, gewonnenen Extrakten erfolgen. Letztere werden bevorzugt, da sie mit gleichbleibender Qualität und gleichbleibender Wirkstoffkonzentration durch Mazeration oder Extraktion mit Wasser, einem niederen Alkohol oder einem solchen Lösungsmittelgemisch hergestellt werden können. Unter Chelidonium majus, d.h. der Pflanze, wird die vor dem Aufblühen oder während der Blütezeit mit der Wurzel gesammelte frische oder getrocknete Pflanze verstanden.

Für die Herstellung des Extraktes von Chelidonium majus kommen in erster Linie die Mazeration in einem ruhenden Lösungsmittel in einem hermetisch verschlossenen Gefäss, die Bewegungsmazeration, wobei der Ansatz durch Rühren, Schütteln oder Wirbeln in Bewegung gehalten wird und die Gegenstromextraktion unter Förderung des Extraktionsgutes mit einer Fördereinrichtung, wie Schnecke, Band oder Schaufel, gegen den Strom des Extraktionsmittels in Frage. Als Extraktionsmittel werden Wasser oder niedere Alkohole, insbesondere Ethanol und Gemische solcher Lösungsmittel, eingesetzt.

Die Wirkstoffkombination kann sowohl systemisch, z.B. peroral oder parenteral, als auch lokal, z.B. als Salben, Cremes, Geles oder Tinktur, angewendet werden, wobei bei Herpes simplex unter lokaler Anwendung vorteilhaftere Wirkungen erzielt wurden. Die Formulierung der jeweiligen Verarbeitungsform kann nach üblichen Methoden unter Verwendung üblicher Mittel und Hilfsstoffe erfolgen.

Die Erfindung wird nun anhand der nachfolgenden Untersuchungen bzw. Beispiele weiter veranschaulicht.

*Beispiel 1*

*Extraktionsverfahren (Perkolation)*

22 g Herba Chelidonii werden mit 300 ml 94%igem Ethanol extrahiert. Das Extraktionsgut ruht in einem zylindrischen Einkörper-Feststoffextraktor auf einem filterbespannten Siebboden auf dem Extraktauslauf und wird vom Extraktionsmittel zunächst durchfeuchtet.

Nach einer 1stündigen Einwirkungszeit wird unter ständiger Zugabe von neuem Extraktionsmittel, unter Berücksichtigung der Durchlaufgeschwindigkeit, bis zur völligen Erschöpfung des Gutes extrahiert.

Der Extrakt wird anschliessend im Wasserbad unter schwachem Vakuum auf 350 ml eingeengt.

1 ml Pflanzenextrakt

| | |
|---|---|
| Chelidonin | 550 bis 600 µg |
| Chelerythrin | 20 bis 30 µg |
| α-Homochelidonin | 10 bis 15 µg |
| Sanguinarin | 200 bis 250 µg |
| Gesamt-Phenanthridin-alkaloide | 1,47 bis 1,16 mg/ml |

der Formeln:

1. Chelidonin $C_{20}H_{19}NO_5$

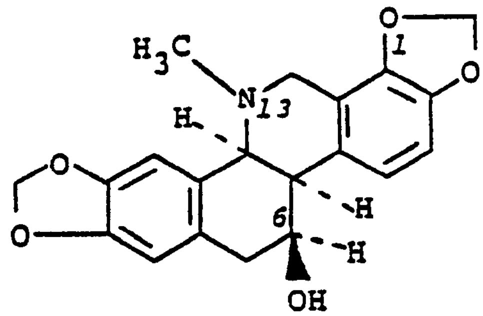

2. Chelerythrin (Toddalin) $C_{21}H_{18}NO_4^+$

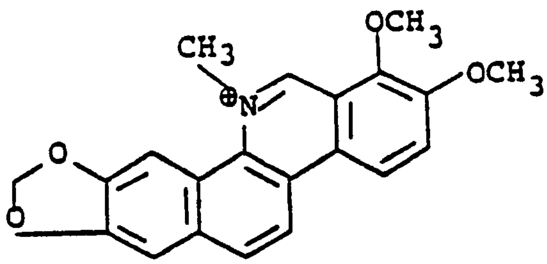

3. Homochelidonin (α–) $C_{21}H_{23}NO_5$

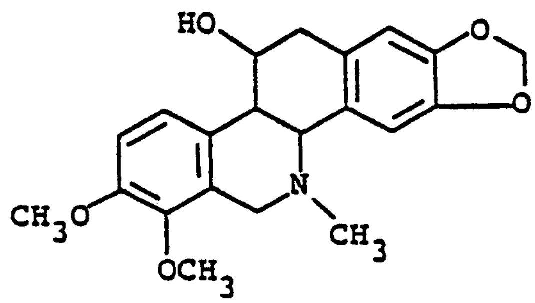

4. Sanguinarin (Pseudochelerythrin) $C_{20}H_{14}NO_4^+$

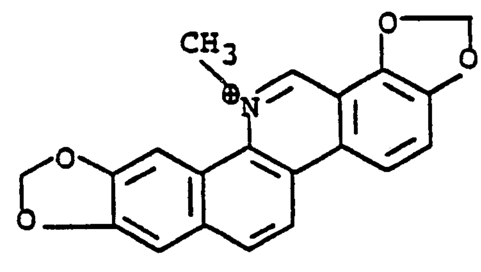

Weitere Extraktionsverfahren werden von Sucher H., et al: Pharmazeutische Technologie (G. Thieme-Verlag, Stuttgart, 1978) beschrieben.

Die Identifizierung der Alkanoide und ihre quantitative Bestimmung erfolgt unter Verwendung von bekannten Vergleichssubstanzen nach bekannten Methoden der Acidimetrie und der direkten Dünnschichtchromatographie.

Für die nachfolgenden Untersuchungen wird das im Beispiel 1 gewonnene Extrakt zur Trockne eingedampft. Diese Trockensubstanz wird dann in DMSO (Dimethylsulfoxid), Methylcellulose oder Tylose zu einer Stammlösung, enthaltend 3 mg trockenes Extrakt pro ml Lösung, gelöst.

*Untersuchungen:*

1. *In vitro Untersuchungen der virostatischen Aktivität des Extraktes von Chelidonium majus*

Diese Untersuchung wurde mit den folgenden Virusstämmen durchgeführt:

Virus Sendai,
reproduziert in 11 Tage altem Hühnerembryo.

Influenzaviren, Typ A-O-58/74/$H_3N_2$/
reproduzierbare Passagen in der Ammionhöhle vorbebrüteter Hühnerembryonen.

Virus vesicular stomatitis (VSV), Stamm Indiana,
reproduzierbar in Gewebekultur L 929.

Herpes simplex, Typ 1 (HSV-1),
reproduzierbar in Affennierenepithelkulturen (GMK).

Virus encephalomyocarditis (EMC), (ColMM),
reproduzierbar in Gewebekultur L 929.

Die Stammlösung, enthaltend 3 mg getrocknetes Extrakt in DMSO, wird mit Eagle's Kulturenlösung mit Zusatz von 2% Kälberserum 1:10, 1:20 und 1:40 verdünnt und mit den einzelnen Virus-Typen 1:1 vermischt.

Verwendet werden Infektionsdosen der Viren von 1000 bis 10 000 (TCID 50). Die Bestimmung des Virustiters im Neutralisationstest erfolgt nach einer Inkubationszeit von 2 Stunden bei Zimmertemperatur (22°C) in 11 Tagen vorbebrüteten Hühnerembryonen bzw. in Gewebekulturen je nach Virusart. Die Resultate dieser in vitro Untersuchungen sind in den Tabellen 1 bis 5 zusammengestellt.

TABELLE 1

*Einfluss des Extraktes von Chelidonium majus auf Virusreplikation in vitro*

| Verdünnung der Stammlösung | Virustiter (1000 TCID 50) (1000 infectious doses) | | | | |
|---|---|---|---|---|---|
| | Sendai | Col MM | VSV | Influenza | Herpes simplex Typ 1 |
| Kontrolle | $10^{6.5}$ | $10^{4.5}$ | $10^{3.5}$ | $10^{3.5}$ | $10^4$ |
| 1:10 | $10^{6.5}$ | $10^{4.5}$ | <10 | $10^{3.5}$ | <10 |

TABELLE 2

*Virustiter in Abhängigkeit von der Konzentration des Extraktes von Chelidonium majus*

| Verdünnung der Stammlösung | VIRUSTITER | |
|---|---|---|
| | Herpes simplex Typ 1 | Virus vesicular stomatitis |
| 1:10 | <10 | <10 |
| 1:20 | <10 | $10^2$ |
| 1:40 | $10^{2.5-3.0}$ | $10^3$ |
| 1:80 | $10^4$ | $10^{3.5}$ |
| Kontrolle | $10^4$ | $10^{3.5}$ |

TABELLE 3

*Einfluss des Extraktes von Chelidonium majus auf die Replikation des Virus vesicular stomatitis (VSV) (Ausgangstiter des Virus $10^6$)*

| Verdünnung der Stammlösung | Hemmung der Replikation des Virus in der Konzentration | | | | |
|---|---|---|---|---|---|
| | $10^{-4}$ | $10^{-5}$ | $10^{-6}$ | $10^{-7}$ | $10^{-8}$ |
| 1:20 | 0 | 0 | 0 | 0 | 0 |
| 1:40 | 0 | 0 | 0 | 0 | 0 |
| 1:80 | +/0 | 0 | 0 | 0 | 0 |
| Kontrolle | + | + | + | 0 | 0 |

+ = zytopathischer Effekt
O = kein zytopathischer Effekt

TABELLE 4

*Einfluss des Extraktes von Chelidonium majus auf die Replikation des Virus ColMM (Ausgangstiter des Virus $10^6$)*

| Verdünnung der Stammlösung | Hemmung der Replikation des Virus in der Konzentration | | | | | |
|---|---|---|---|---|---|---|
| | $10^{-3}$ | $10^{-4}$ | $10^{-5}$ | $10^{-6}$ | $10^{-7}$ | $10^{-8}$ |
| 1:20 | + | + | + | + | + | 0 |
| Kontrolle | + | + | + | + | + | 0 |

+ = zytopathischer Effekt
* = kein zytopathischer Effekt

TABELLE 5

*Einfluss des Extraktes von Chelidonium majus auf die Replikation des Virus Sendai (Ausgangstiter des Virus $10^6$)*

| Verdünnung der Stammlösung | Hemmung der Replikation des Virus in der Konzentration | | | | | | |
|---|---|---|---|---|---|---|---|
| | $10^{-3}$ | $10^{-4}$ | $10^{-5}$ | $10^{-6}$ | $10^{-7}$ | $10^{-8}$ | $10^{-9}$ |
| 1:20 | + | + | + | + | +/0 | +/0 | +/0 |
| Kontrolle (DMSO) | + | + | + | + | +/0 | +/0 | +/0 |
| Kontrolle (in PBS) | + | + | + | + | + | 0 | 0 |

+ = zytopathischer Effekt
0 = kein zytopathischer Effekt
DMSO = Dimethylsulfoxid
PBS = Phosphatpuffer/pysiologische Salzlösung

2. *Untersuchung der virostatischen Wirkung des Extraktes von Chelidonium majus mittels Plaque-Assay*

Platten von 35 mm Durchmesser werden mit einer HeLa-Zellkultur (Flow) in einer Menge von $0,5 \times 10^6$ Zellen/Platte beschichtet. Auf die zu einschichtigen Zellrasen, sog. Monolayers, auswachsenden Zellkulturen wird eine verdünnte Suspension von Vakziniavirus bzw. Herpes simplex, Typ 1 und 2 mit einem Titer von $10^4$ $TCID_{50}$ in einer Menge von 0,3 ml/Platte gegeben.

Die Zellkulturen werden während 1 Stunde bei 37°C in $CO_2$-Atmosphäre inkubiert, bis die Stadien der Adsorption und der Penetration abgelaufen sind. Dann giesst man die überschüssige Kulturflüssigkeit ab und überschichtet den infizierten Rasen mit 3 ml 0,5% noch flüssigem Agar (Agar Difco mit 5% fötalem Kälberserum und Eagle's minimal essential medium).

Nach dem Erstarren des Agars, nach ca. 1 Stunde bei Zimmertemperatur, wird auf jede Platte ein Filterpapierplättchen mit einem Durchmesser von 10 mm gelegt, das die Stammlösung in 15% DMSO, enthaltend 3 mg Trockenextrakt/ml, in einer Verdünnung von 1:150 enthält. Anschliessend werden Verdünnungen von 1:150 bis 1:4500 geprüft.

Auf Kontrollplättchen wird das Lösungsmittel, d.h. eine 15%-DMSO-Lösung, aufgebracht.

Anschliessend werden die Platten während 24 Stunden in $CO_2$-Atmosphäre bei 37°C inkubiert (Assab). Nach der Inkubation wird der Zellrasen mit 5% Neutralrot in Agar (3 ml/Platte) überschichtet und nochmals während 72 Stunden in $CO_2$-Atmosphäre bei 37°C bebrütet. Die Beurteilung erfolgt mikroskopisch bei 20facher Vergrösserung. Man bezeichnet die Virusmenge, die ausreicht, um einen Plaque zu erzeugen, als Plaque Forming Unit.

Die Zellen, die vom Virus befallen sind, können nicht mehr angefärbt werden. Sie erscheinen dann als farblose Flecken (Plaques) innerhalb des gefärbten Rasens der Normalzellen. Die Hemmung der Plaque-Bildung wird als Hemmhof in mm angegeben. Je grösser der Hemmhof, desto besser die virostatische Aktivität des Extraktes. Die Grösse der ungefärbten ringförmigen Aussparung am Rande des Plättchens ist ein Mass für die zytotoxische Aktivität des Extraktes (G.May und D. Peteri, Arzneimittelforschung 23; 5, 1973; Rada und Zavada, Neoplasma 9; 1, 1962).

*Resultate mit Vakziniavirus:*

Virusinfizierte Kontrollplatten zeigen deutliche farblose Flecken im Bereich von virusbefallenen Zellen auf der ganzen Plattenoberfläche. Auf den mit dem Extrakt behandelten Platten lassen sich lediglich vereinzelte ungefärbte Infektionsherde an der Peripherie des Papierplättchens feststellen. Nahezu die

gesamte Plattenoberfläche zeigt einen gleichmässigen Rasen virusunbeschädigter HeLa-Zellen.

*Resultate mit Herpes simples, Typ 1 und 2:*

Im Plaque-Test zeigt das Extrakt eine deutliche virostatische Aktivität in einer Verdünnung von 1:150 bis 1:4500.

Die Resultate sind in der Tabelle 6 zusammengestellt.

TABELLE 6

*Einfluss des Extraktes von Chelidonium majus auf Vaccinia- und Herpes simplex-Virus (Typ 1 und Typ 2) im Plaque-Test*

| Verdünnung der Stammlösung | Hemmhof (mm) | | | | | |
|---|---|---|---|---|---|---|
| | Vaccinia | | Herpes simplex Typ 1 | | Herpes simplex Typ 2 | |
| | Wirkung | | | | | |
| | virostatisch | zytotoxisch | virostatisch | zytotoxisch | virostatisch | zytotoxisch |
| 1:150 | 15;11;⩾12; ⩾12 | 2;0;0;0 | 13;18;11;15; ⩾12 | 1;0;1;0;0 | 15;13;10;12;9 | 0;0;1;0;0 |
| 1:1500 | 3;3;5;0 | 2;0;0;0 | 4;5;6;5 | 1;0;0;0 | 5;4;3;4;2 | 0;0;1;0;0 |
| 1:4500 | 0;0;0;0 | 2;0;0;0 | 0;2;2;1 | 1;0;0;0 | 2;2;0;1;0 | 0;0;0;0;0 |
| Kontrolle 15% DMSO | 0;0;0;0 | 2;0;0;0 | 0;0;0;0 | 1;0;0;0 | 0;0;0;0;0 | 0;0;0;0;0 |

3. *Untersuchung der Induktion der Interferonproduktion*

Untersuchungen in einer Leukozyten-Suspension in vitro zeigen bei einer Verdünnung der Stammlösung von 1:20, 1:50, 1:100, 1:200 und 1:300 keinen Induktion der Interferonproduktion. Bei der Verwendung von Sendai-Virus wird kein Einfluss auf den Virusinduktor beobachtet.

4. *Untersuchung der Wirkung des Extraktes auf Herpes simplex-Infektion der Haut*

Die Wirkung des Extraktes von Chelidonium majus wird, in Anlehnung an die Methode von Huber et al, J. invest. Dermatol, 62, 92 bis 95, 1974, auf eine Herpes simplex-Infektion der Haut bei männlichen Meerschweinchen, Typ Albino von 200 bis 350 g Gewicht, untersucht.

Um mögliche Hautverletzungen auszuschliessen, wird die Rückenhaut der Tiere 48 Stunden vor dem Beginn der Untersuchung geschoren. Anschliessend werden die Tiere in Gruppen zu je 10 Tieren eingeteilt. Im Bereich der depilierten Rückenhaut werden nach vorheriger Reinigung mit Äther vier Felder markiert und je nach Tiergruppe wird Herpes simplex Typ 1 (HSV-1) bzw. Typ 2 (HSV-2) 0,05 ml Virussuspension in einer Menge von $10^7$ PFU/ml (Plaque-Forming Unit = PFU) auf die markierten Flächen aufgetragen. Nach dem Auftragen wird die Virussuspension durch zwanzig oberflächliche Einstiche mit einer Injektionsnadel auf einer Fläche von ca. 1 $cm^2$ inokuliert. Der Rest der Virussuspension wird sorgfältig in die Haut eingerieben. 0,1 ml der Stammlösung in Methylcellulose wird in drei verschiedenen Verdünnungen (1%, 5% und 10%) auf die entsprechenden Herpes-Virus-induzierten Hautläsionen im Bereiche der Feldflächen 2mal täglich aufgetragen. Das vierte Kontrollfeld wird mit Methylcellulose allein behandelt.

Gleichzeitig wird eine Kontrollgruppe gebildet, um den Verlauf der Virusinfektion bei unbehandelten Tieren zu verfolgen.

Die auf diese Weise induzierten herpetischen Hautläsionen, das Ausmass der Hautschädigung und des Heilungsverlaufes werden während 14 Tagen beobachtet.

Die Beurteilung der herpetischen Hautschädigung erfolgt nach folgendem Bewertungsschema:

| *Punktzahl* | *Hautdefekt* |
|---|---|
| 1 | Erythem und leichtes Ödem; |
| 2 | Erythem (Hyperämie), Ödem, Papeln und Vesikeln; |
| 3 | Papeln, Vesikeln und Auftreten von Erosionen; |
| 4 | konfluierende Erosionen, stellenweise Epithelisation; |
| 5 | wie oben, jedoch mit der Tendenz zum Übergreifen der Läsionen auf die Umgebung. |

Bei den Kontrolltieren treten die Hautveränderungen in folgender Reihenfolge auf:

| | |
|---|---|
| 1. Tag: | Erythem und leichtes Ödem; |
| 2. bis 3. Tag: | Papeln und Vesikeln; |
| 4. bis 8. Tag: | Auftreten von Erosionen und Krusten; |
| 9. bis 14. Tag: | Spontane Rückbildung der Hautläsionen. |

Es hat sich gezeigt, dass die 5%- und 10%-Verdünnung der Stammlösung die HSV-2-induzierten Hautläsionen beim Meerschweinchen signifikant hemmen. In der Regel waren in dieser Gruppe im Bereich der behandelten Areale keine Bläscheneruptionen sichtbar. Es liess sich lediglich, ausser einem lokalen Erythem, eine tastbare Infiltration der Haut feststellen. Später trat eine Desquamation auf. Unter der Behandlung war die Dauer dieser abortiven Veränderungen deutlich verkürzt. Die Heilung der Hautveränderungen war nach einer Beobachtungszeit von 8 bis 10 Tagen praktisch immer vollständig.

Die 1%ige Verdünnung der Stammlösung war nur schwach wirksam. Im Bereich der behandelten Flächen fanden sich Papeln und Bläschen, die jedoch keine Konfluenztendenz zeigten. Obwohl die Krustenbildung relativ rasch auftrat, war die Heilung von Hautläsionen mit einer Dauer von 14 Tagen nicht signifikant verkürzt.

Das Extrakt von Chelidonium majus erwies sich bei HSV-1-induzierten Hautveränderungen im Meerschweinchenmodell als weniger wirkungsvoll. Dermale Applikation von 5%- und 10%iger Verdünnung der Stammlösung vermochte das Auftreten von Erythem, Ödem und vereinzelten Papulovesikeln nicht zu unterdrücken. Allerdings war das Ausmass der Bläscheneruption deutlich vermindert. Erosionen traten nur sporadisch auf und zeigten rasche Epithelisation. Die 5%- und 10%ige Verdünnung verkürzte die Abheilzeit der herpetischen Läsionen auf 10 Tage.

Bei der Verwendung der 1%igen Verdünnung der Stammlösung liess sich keine therapeutische Wirkung feststellen.

In der HSV-2-infizierten Gruppe konnte im Anschluss an die Inokulation kein Sterben der Tiere beobachtet werden.

Hingegen sind in der HSV-1-infizierten Gruppe einige Tiere (1-3) an neurologischen Komplikationen gestorben. Dies trifft sowohl auf die 5%/10%ige Verdünnung als auch auf die unbehandelte Kontrollgruppe zu.

Die Resultate dieser Untersuchungen sind in Tabelle 7 und 8 zusammengestellt.

TABELLE 7

*Einfluss des Extraktes von Chelidonium majus auf die Entwicklung der Hautläsionen bei Herpes simplex Typ 1-Infektion (HSV-1) (Mittelwerte)**

| Verdünnung der Stammlösung | 1. Tag | 2.-3.Tag | 4.-8.Tag | 9.-14.Tag |
|---|---|---|---|---|
| 1% | 2 | 2.5→4 | 5 | 5→1.5 |
| 5% | 1 | 2→2.5 | 4 | 1 |
| 10% | 1 | 2 | 3 | 1.5 |
| Kontrolle | 1.5 | 2→3.5 | 5 | 5→1 |

*) Die Bewertung der Reaktion erfolgte nach einem Beurteilungsschema (Punktzahl).

TABELLE 8

*Einfluss des Extraktes von Chelidonium majus auf die Entwicklung der Effloreszenzen bei Herpes simplex Typ 2-Infektion (HSV-2) (Mittelwerte)*

| Verdünnung der Stammlösung | 1. Tag | 2.-3.Tag | 4.-8.Tag | 9.-14.Tag |
|---|---|---|---|---|
| 1% | 1.2 | 3 | 5 | 5→1.5 |
| 5% | 1 | 1.5→3 | 3→1.5 | 1 |
| 10% | 1.5 | 2 | 2→1 | 1 |
| Kontrolle | 1.5 | 2→3 | 5 | 5→1 |

5. *Untersuchung der Wirkung des Extraktes auf Herpes simplex-Infektion am Mäuseohr*

Die Untersuchung wird an 3 bis 4 Wochen alten Mäusen beiderlei Geschlechtes, Stamm Balb/c, durchgeführt. Das Mäuseohr wird mit einer Pinzette gefasst und auf die Haut der Innenseite wird ein Tropfen der Virussuspension, HSV-1 bzw. HSV-2 (Stamm $MB_2$), in einer Menge von $10^7$ PFU/ml aufgetragen. Anschliessend werden durch das Inokulum mit einer dünnen Injektionsnadel zwanzig oberflächliche Einstiche vorgenommen, wobei die Injektionsnadel tangential gehalten wird, um Blutungen zu vermeiden. Die Virusinokula werden somit intradermal injiziert.

Zur Prüfung der lokalen Wirksamkeit des Extraktes wird nach der Inokulation am rechten Ohr 2mal täglich eine Verdünnung der Stammlösung von 1:10 bzw. 1:20 in Tylose appliziert. An der kontralateralen Stelle am linken Ohr wird als Kontrolle nur Tylose aufgetragen. Bei einer Kontrollgruppe ohne Behandlung wird der Verlauf der experimentellen Herpesinfektion parallel registriert.

Zur Prüfung der systemischen Wirksamkeit des Extraktes wird nach dem Infizieren eine Verdünnung der Stammlösung von 1:10 bzw. 1:20 in Tylose ($LD_{50}$) 2mal täglich intraperitoneal verabreicht. Bei den Kontrollen wird nur Tylose als Lösungsmittel intraperitoneal injiziert. Eine zweite Kontrollgruppe wird nicht behandelt.

Bewertet werden die lokalen klinischen Veränderungen, die der menschlichen Herpes simplex-Erkrankung ähneln und die Überlebensrate der Tiere jeden 2. Tag, beginnend am 6. Tag post infectionem.

Die Beurteilung der lokalen Herpes simplex-induzierten Veränderungen wird nach folgendem Schema bewertet:

| *Punktzahl* | *Hautdefekt* |
|---|---|
| 1 | Erythem und Hyperämie; |
| 2 | Hyperämie, Erythem, kleine Erosionen und vereinzelte Bläschen; |
| 3 | Hyperämie, Erythem, zahlreiche Bläschen, Erosionen, Krusten; |
| 4 | wie oben, zusätzlich nekrotische Veränderungen. |

6 Tage nach Auftragen des Inokulums wurde in den Kontrollgruppen das Auftreten von Erythem (Hyperämie) und Ödem festgestellt. Im weiteren Verlauf

der experimentellen Virusinfektion wurden Bläscheneruptionen, Erosionen, Krusten und manchmal sogar umschriebene nektrotische Veränderungen im Bereiche der Ohrinnenseite beobachtet.

Das Extrakt beeinflusst in signifikanter Weise den Ablauf der experimentellen HSV-1- und HSV-2-Infektion am Mäuseohr.

Eine dermale oder systemische Applikation des verdünnten Extraktes bewirkt eine Verminderung des lokalen Ödems und des Ausmasses der Bläscheneruption. Die wenigen Bläschen zeigten auch keine Tendenz zur Konfluenz. Erosionen und Krusten waren deutlich weniger ausgeprägt.

Im Laufe der Untersuchung starben die Tiere an Herpes simplex-induzierten Veränderungen des Zentralnervensystems sowohl in der Kontrollgruppe als auch in jener Extraktgruppe, deren Tiere intraperitoneal behandelt wurden. Erstaunlicherweise überlebten alle virusinfizierten Tiere, welche mit dem Extrakt mittels lokaler Applikation behandelt wurden, während bei der intraperitonealen Applikation 40 bis 60% der Tiere an Herpes simplex-induzierten Komplikationen starben.

Die Resultate sind in den Tabelle 9 bis 12 zusammengestellt.

TABELLE 9

*Einfluss des lokal verabreichten Extraktes von Chelidonium majus auf die HSV-1-Infektion am Mäuseohr*

| Verdünnung der Stammlösung | Tage post infectionem | | | | | | | | | | | | | |
|---|---|---|---|---|---|---|---|---|---|---|---|---|---|---|
| | 6 | | 8 | | 10 | | 12 | | 14 | | 16 | | 18 | |
| | L | R | L | R | L | R | L | R | L | R | L | R | L | R |
| 1:20 | 1.0 | 0.7 | 1.6 | 0.9 | 1.5 | 0.9 | 0.9 | 0.7 | 0.2 | 0.2 | 0.1 | 0.1 | 0 | 0 |
| 1:10 | 1.1 | 0.5 | 1.5 | 0.8 | 1.6 | 0.8 | 2.0 | 1.5 | 1.3 | 0.7 | 0.3 | 0.1 | 0.1 | 0 |
| Virus allein | 2.3 | | 2.2 | | 1.6 | | 2.1 | | 1.5 | | 1.1 | | 0.6 | |

L = linkes Ohr, Tylose
R = rechtes Ohr, Extrakt

TABELLE 10

*Einfluss des systemisch verabreichten Extraktes von Chelidonium majus auf die HSV-1-Infektion am Mäuseohr*

| Verdünnung der Stammlösung | Tage post infectionem | | | | | | |
|---|---|---|---|---|---|---|---|
| | 6 | 8 | 10 | 12 | 14 | 16 | 18 |
| 1:20 | 0.5 | 1.2 | 1.6 | 1.9 | 1.5 | 0.8 | 0 |
| 1:10 | 0.7 | 0.9 | 0.6 | 0.6 | 0.2 | 0 | 0 |
| HSV-1 + Tylose Kontrolle | 1.7 | 1.8 | 1.4 | 1.8 | 1.2 | 0.8 | 0.6 |
| HSV-1 allein Kontrolle | 2.3 | 2.2 | 1.6 | 2.1 | 1.5 | 1.1 | 0.6 |

TABELLE 11

*Einfluss des lokal verabreichten Extraktes von Chelidonium majus auf die HSV-2-Infektion am Mäuseohr*

| Verdünnung der Stammlösung | Tage post infectionem | | | | | | | | | | | | | |
|---|---|---|---|---|---|---|---|---|---|---|---|---|---|---|
| | 6 | | 8 | | 10 | | 12 | | 14 | | 16 | | 18 | |
| | L | R | L | R | L | R | L | R | L | R | L | R | L | R |
| 1:20 | 2.0 | 1.7 | 2.1 | 1.2 | 2.2 | 1.4 | 2.0 | 1.6 | 1.4 | 1.0 | 0.5 | 0.3 | 0.2 | 0 |
| 1:10 | 2.4 | 1.1 | 2.4 | 1.0 | 2.6 | 1.2 | 2.3 | 1.5 | 1.0 | 0.6 | 0.5 | 0.1 | 0 | 0 |
| Virus allein | 3.1 | | 2.8 | | 2.3 | | 1.7 | | 1.2 | | 0.9 | | 0.3 | |

L = linkes Ohr, Tylose
R = rechtes Ohr, Extrakt

TABELLE 12

*Einfluss des systemisch verabreichten Extraktes von Chelidonium majus auf die HSV-2-Infektion am Mäuseohr*

| Verdünnung der Stammlösung | Tage post infectionem | | | | | | |
|---|---|---|---|---|---|---|---|
| | 6 | 8 | 10 | 12 | 14 | 16 | 18 |
| 1:20 | 1.4 | 1.7 | 1.7 | 1.5 | 1.0 | 0.6 | 0.4 |
| 1:10 | 1.0 | 1.3 | 0.8 | 0.8 | 0.4 | 0.2 | 0.2 |
| HSV-1 + Tylose Kontrolle | 2.7 | 2.6 | 2.3 | 1.4 | 0.9 | 0.6 | 0.4 |
| HSV-1 allein Kontrolle | 3.1 | 2.8 | 2.3 | 1.7 | 1.2 | 0.9 | 0.3 |

6. *Untersuchung der Wirkung des Extraktes auf Herpes-Keratitis (HSV-1) an Kaninchen-Kornea*

Durch die Inokulation von Herpes simplex-Virus Typ 1 in einer Infektionsdosis von $10^4$ $TCID_{50}$ wird beim Kaninchen eine experimentelle Herpes-Keratitis induziert.

Vor der Inokulation wird die Kornea mit einem Baumwolle-Tupfer gereinigt und mit einem Tropfen der unverdünnten Virussuspension infiziert. Die Herpes-Keratitis wird an beiden Augen ausgelöst. Das linke Auge gilt als Kontrolle und wird anschliessend mit zwei Tropfen 2%iger Methylcellulose 2mal täglich behandelt. Von mit Methylcellulose 1:10 und 1:20 verdünnter Stammlösung werden 2mal täglich je zwei Tropfen in das rechte Auge instilliert. Die Behandlung beginnt am Tag der Virusinokulation. Die Beurteilung der Reaktion erfolgt vom 2. Tag nach der Virusinokulation an, jeden 2. Tag, nach einem festgelegten Punkte-Score-System.

Als Kontrolle werden mehrere Tiere gleichzeitig mit dem Virus infiziert und der Verlauf der lokalen Reaktion bewertet. Gleichzeitig wird die okulare Verträglichkeit des verdünnten Extraktes beim Kaninchen geprüft.

Die Applikation des Extraktes von Methylcellulose auf das Auge des Kaninchens hat keine nachweisbare Unverträglichkeitsreaktion, weder auf das Auge noch auf seine Adnexe, zur Folge gehabt. Die Resultate werden in der Tabelle 13 dargestellt.

Zwischen dem 14. und 21. Tag betrug die Mortalität bei den mit dem Extrakt behandelten Tieren 50%. Bei ausschliesslich mit Virus infizierten Tieren betrug die Mortalität in der gleichen Beobachtungsperiode 100%.

*Bewertungsschema am Kaninchenauge:*

| *Punktzahl* | *Befund* |
|---|---|
| 1 | Geringgradige Rötung der Konjunctiva bulbi ohne Ödem; geringgradige Exsudation. |
| 2 | Rötung der Konjunctiva bulbi mit Ödem und geringgradiger Injektion und Exsudation. |
| 3 | Starke purulent-schleimige Exsudation; starke Rötung der Konjunctiva bulbi mit deutlicher Injektion; Ödem; corneale Vesikeln. |
| 4 | Wie oben + Bläschen + Krustenbildung. |
| 5 | Wie oben; ganz geschlossene Lider. |

TABELLE 13

*Einfluss des Extraktes von Chelidonium majus auf HSV-1-Keratitis*

| Verdünnung der Stammlösung | Bewertung der lokalen Reaktion | | | | | | | | | | | | | | | | | |
|---|---|---|---|---|---|---|---|---|---|---|---|---|---|---|---|---|---|---|
| | 2.Tag | | 4.Tag | | 6.Tag | | 8.Tag | | 10.Tag | | 12.Tag | | 14.Tag | | 16.Tag | | 18.Tag | |
| | L | R | L | R | L | R | L | R | L | R | L | R | L | R | L | R | L | R |
| 1:20 (R) Methylcellulose 2% (L) | 3.25 | 2.8 | 3.5 | 2.5 | 3.0 | 2.8 | 1.0 | 1.0 | 1.0 | 0.7 | 0.5 | 0.5 | 0 | 0 | 0 | 0 | 0 | 0 |
| 1:10 (R) Methylcellulose 2% (L) | 3.8 | 2.7 | 4.4 | 3.2 | 4.2 | 2.5 | 2.9 | 2.0 | 2.5 | 1.6 | 2.4 | 1.8 | 1.2 | 1.2 | 1.5 | 1.5 | 1.3 | 1.3 |
| HSV-1 allein Kontrolle | 3.7 | | 3.3 | | 4.0 | | 3.5 | | 3.0 | | 2.6 | | 1.4 | | 1.4 | | 1.2 | |

L = linkes Auge
R = rechtes Auge

Die Toxizität des Extraktes ist bereits bekannt, so dass sie nicht untersucht worden ist.

Wie aus den obigen Untersuchungen hervorgeht, beruht die Wirkung der im Extrakt enthaltenen Wirkstoffkombination auf einer spezifischen Hemmung der Virusreplikation. Dabei wird allgemein eine schneller eintretende Heilung als mit bekannten Mitteln erzielt.

Die Beispiele 2 bis 5 veranschaulichen verschiedene Ausführungsformen des erfindungsgemässen Mittels.

*Beispiel 2*

Extractum Herb. Chelidonii: 10 ml.
Alkoholischer Auszug (Äthanol 70%) aus Herba Chelidonii 1:10; entsprechende Gesamt-Phenanthriolinalkaloide 1,47 bis 1,61 mg/ml.

*Beispiel 3*

Extractum Herb. Chelidonii spir. sicc.
Extracta sicca durch Extraktion mit 70 bzw. 90% Ethanol (gemäss Pharmakopea helv.).

*Beispiel 4*

Herbae Chelidonii pulv.
Lanolini
Vaselini aa 10,0
Zu Salbe gemischt

*Beispiel 4*

Tinctura Chelidonii 5,0
Adipis lanae ad 100,0
Zu Salbe gemsicht

**Patentansprüche**

1. Verwendung eines Extraktes der Pflanze Chelidonium majus in Wasser, einem niederen Alkohol oder einem Gemisch solcher Lösungsmittel zur Herstellung eines therapeutischen Mittels zur Bekämpfung von durch Herpesviren verursachten Infektionen.

2. Verwendung eines Wasser-Ethanol-Extraktes nach Anspruch 1.

3. Verwendung einer Mischung von Alkaloiden des Benzophenanthridin-Typus aus Chelidonium majus zur Herstellung eines therapeutischen Mittels zur Bekämpfung von durch Herpesviren verursachten Infektionen.

4. Verwendung nach Anspruch 3, dadurch gekennzeichnet, dass Chelidonin, Chelerythrin und Sanguinarin und/oder deren Derivate verwendet werden.

**Claims**

1. Use of an extract of the plant Chelidonium majus in water, a lower alcohol or a mixture of such solvents, for the preparation of a therapeutic agent for controlling infections caused by herpes viruses.

2. Use of a water/ethanol extract according to Claim 1.

3. Use of a mixture of alkaloids of the benzophenanthridine type of Chelidonium majus (sic) for the preparation of a therapeutic agent for controlling infections caused by herpes viruses.

4. Use according to Claim 3, characterized in that chelidonin, chelerythrin and sanguinarin and/or their derivatives are used.

**Revendications**

1. Utilisation d'un extrait de la plante Chelidonium majus dans l'eau, un alcool inférieur ou un mélange de tels solvants pour la préparation d'un agent thérapeutique pour la lutte contre les infections provoquées par des herpès-virus.

2. Utilisation d'un extrait-eau-éthanol selon la revendication 1.

3. Utilisation d'un mélange d'alcaloïdes du type benzophénanthridine provenant de Chelidonium majus pour la préparation d'un agent thérapeutique pour la lutte contre les infections provoquées par des herpès-virus.

4. Utilisation selon la revendication 3, caractérisée en ce que l'on utilise de la chélidonine, de la chélérythrine et de la sanguinarine et/ou leurs dérivés.